# EUROPÄISCHE PATENTANMELDUNG

(11) **EP 1 313 065 A1**
(43) Veröffentlichungstag der Anmeldung: **21.05.2003**
(21) Anmeldenummer: 02102588.7
(22) Anmeldetag: 14.11.2002
(51) Int. Cl.: G06T 5/00

(54) **Verfahren und Vorrichtung zur Kalibrierung von und zur Bilderzeugung mit einer schwerkraftempfindlichen Röntgenaufnahmeeinrichtung**

(30) Priorität: 16.11.2001 DE 10156445
(71) Anmelder: Philips Intellectual Property & Standards GmbH, 20099 Hamburg (DE); Koninklijke Philips Electronics N.V., 5621 BA Eindhoven (NL)
(72) Erfinder: Sabczynski, Jörg, Dr., 52088 Aachen (DE); Zylka, Waldemar, Prof. Dr., 52088 Aachen (DE)
(74) Vertreter: Volmer, Georg, Dipl.-Ing.

(57) **Zusammenfassung**

Es wird ein Verfahren und eine Vorrichtung zur Kalibrierung einer Bildaufnahmeeinrichtung beschrieben, die z B. aufgrund ihres Einbaus in eine ausladende Tragekonstruktion schwerkraftempfindlich ist, d h bei der durch mechanische Deformationen der Tragekonstruktion die Bildgeometrie beeinflusst und insbesondere Bildverzerrungen verursacht werden können. Es wird weiterhin ein Verfahren und eine Vorrichtung zur Bilderzeugung mit einer solchen Bildaufnahmeeinrichtung beschrieben, wie sie insbesondere in Röntgensystemen, z. B. solchen mit C-Arm, Anwendung findet. Die Kalibrierung erfolgt im wesentlichen durch Erstellen und Abspeichern einer Nachschlagstabelle, mit der einer Mehrzahl von Positionsdaten der Tragskonstruktion die zur Korrektur der dadurch verursachten Verzerrungen notwendigen Kalibrierungsdaten zugeordnet werden. Bei der Bilderzeugung werden dann bei der Aufnahme eines Bildes die Richtung der Schwerkraft relativ zu der Tragekonstruktion gemessen, daraus die Positionsdaten berechnet und die diesen Daten in der Tabelle zugeordneten Kalibrierungsdaten ausgelesen und zur Korrektur des aufgenommenen Bildes verwendet.

## Beschreibung

Die Erfindung betrifft ein Verfahren und eine Vorrichtung zur Kalibrierung einer Bildaufnahmeeinrichtung, die z. B. aufgrund ihres Einbaus in eine ausladende Tragekonstruktion schwerkraftempfindlich ist, d h. bei der durch mechanische Deformationen der Tragekonstruktion die Bildgeometrie beeinflusst und insbesondere Bildverzerrungsn verursacht werden können. Die Erfindung betrifft weiterhin ein Verfahren und eine Vorrichtung zur Bilderzeugung mit einer solchen Bildaufnahmeeinrichtung, wie sie insbesondere in Röntgensystemen, z. B. solchen mit C-Arm, Anwendung findet. Die Erfindung betrifft schließlich auch ein Röntgensystem mit solchen Vorrichtungen.

Bildverzerrungen des genannten Ursprungs treten insbesondere bei ausladenden und beweglichen mechanischen Tragekonstruktionen für solche Bildaufnahmeeinrichtungen durch mechanische Deformationen auf. Dieses Problem zeigt sich in besonderem Maße bei Röntgensystemen, bei denen die Bildaufnahmeeinrichtung an einem um einen Patienten schwenkbaren Arm (C-Arm) befestigt ist, an dessen einem Ende sich die Bildaufnahmeeinrichtung und an dessen anderem Ende sich eine Röntgenstrahlenquelle befindet. Aufgrund der relativ großen Abmessungen des Arms und des relativ hohen Gewichtes dieser beiden Komponenten kann sich der C-Arm in Abhängigkeit von seiner Drehstellung in unterschiedlichem und so starkem Maße deformieren, dass die aufgenommenen Bilder verzerrt sind.

Aus der WO 00/66971 ist eine Vorrichtung zur Erfassung der Position und Orientierung eines Körpers mit Hilfsmitteln zur Ermittlung der Richtung des Schwerkraftvektors, sowie ein Verfahren zur Korrektur von Röntgenbildern, die durch die Schwerkraft und das Erdmagnetfeld verzerrt sind, offenbart. Dabei werden zur Korrektur eines aufgenommenen und digitalisierten Röntgenbildes zunächst die Richtung des Schwerkraftvektors und die Position und Orientierung des Röntgengerätes erfasst und dann die durch die Deformation des Röntgengerätes sowie das lokale Erdmagnetfeld verursachten Verzerrungen des Röntgenbildes ermittelt, um schließlich diese Verzerrungen in dem gespeicherten Röntgenbild mit Hilfe eines Rechners zu korrigieren. Ein wesentlicher Nachteil besteht hierbei jedoch darin, dass zur Bildkorrektur die Vorrichtung zur Bestimmung der genauen Position und Orientierung des Röntgengerätes, sowie gegebenenfalls ein zusätzlicher Rechner erforderlich ist. Dieser zusätzliche apparative Aufwand ist insbesondere bei mobilen Röntgengeräten nachteilig.

Eine Aufgabe, die der Erfindung zugrunde liegt, besteht deshalb darin, ein Verfahren und eine Vorrichtung anzugeben, mit dem /der eine Kalibrierung einer Bildaufnahmeeinrichtung im Hinblick auf Bildverzerrungen der eingangs genannten Art auf relativ einfache und zuverlässige Weise möglich ist.

Mit der Erfindung soll auch ein Verfahren und eine Vorrichtung zu Kalibrierung angegeben werden, das /die insbesondere zur Anwendung in Röntgensystemen mit C-Arm zur Kompensation von Bildverzerrungen geeignet ist, die durch die durch die Schwerkraft verursachten mechanischen Deformationen des C-Arms hervorgerufen werden.

Weiterhin soll ein Verfahren und eine Vorrichtung zur Bilderzeugung angegeben werden, das /die insbesondere zur Anwendung mit einer erfindungsgemäß kalibrierten Bildaufnahmeeinrichtung und optional auch zur chirurgischen Navigation ausgestaltet ist.

Schließlich soll auch ein insbesondere für mobile Anwendungen geeignetes Röntgensystem geschaffen werden, bei dem die eingangs genannten Schwerkrafteinflüsse ohne wesentlichen zusätzlichen apparativen Aufwand, wie zum Beispiel ein Positionsmesssystem, kompensiert werden können.

Gelöst wird diese Aufgabe gemäß Anspruch 1 mit einem Verfahren zur Kalibrierung einer mit einer schwerkraftempfindlichen Tragekonstruktion gehaltenen Bildaufnahmeeinrichtung mit folgenden Schritten: Ermitteln von Kalibrierungsdaten (Kalibrierungs-Basispunkten) in einer Mehrzahl von gewählten Stellungen der Tragekonstruktion, mit denen ein in der jeweiligen Stellung mit der Bildaufnahmeeinrichtung aufgenommenes Bild entzerrt werden kann; Ermitteln von jeweils auf die Richtung der Schwerkraft bezogenen Positionsdaten der Tragekonstruktion in den gewählten Stellungen; und Zuordnen der Positionsdaten zu den Kalibrierungsdaten in einer Nachschlagetabelle.

Die Aufgabe wird gemäß Anspruch 5 mit einer Vorrichtung zur Durchführung des Verfahrens mit folgenden Merkmalen gelöst: eine Einrichtung zum Erfassen der Richtung der Schwerkraft relativ zu einer Stellung der Tragekonstruktion; und eine Rechen- und Speichereinheit zum Ermitteln von auf die Richtung der Schwerkraft bezogenen Positionsdaten für eine Mehrzahl von gewählten Stellungsn der Tragekonstruktion und zum Erstellen und Abspeichern einer Nachschlagetabelle, mit der den Positionsdaten jeweils die zur Entzerrung von in diesen Stellungen durch mechanische Deformationen verursachten Bildverzerrungen geeigneten Kalibrierungsdaten zugeordnet sind

Die Aufgabe wird weiterhin gemäß Anspruch 6 mit einem Verfahren zur Bilderzeugung mit einer mit einer schwerkraftempfindlichen Tragekonstruktion gehaltenen Bildaufnahmeeinrichtung mit folgenden Schritten gelost: Aufnehmen eines Bildes eines Untersuchungsobjektes in einer gewählten Stellung der Tragekonstruktion; Ermitteln von auf die Richtung der Schwerkraft bezogenen Positionsdaten der Tragekonstruktion in der gewählten Stellung; Vergleichen der ermittelten Positionsdaten mit den in einer Nachschlagetabelle gespeicherten Positionsdaten; Auslesen von Kalibrierungsdaten, die unter zumindest im wesentlichen mit den ermittelten Positionsdaten übereinstimmenden Positionsdaten in der Nachschlagetabelle gsspeichert sind; und Entzerren des aufgenommenen Bildes mit den ausgelesenen Kalibrierungsdaten.

Schließlich wird die Aufgabe gemäß Anspruch 9 mit einer Vorrichtung zur Durchführung des Bilderzeugungsverfahrens mit folgenden Merkmalen gelöst: einer Einrichtung zur Erfassung der Richtung der Schwerkraft relativ zu der Tragekonstruktion; einer Rechen- und Speichereinheit zum Ermitteln von auf die Richtung der Schwerkraft bezogenen Positionsdaten der Tragekonstruktion, zum Vergleichen dieser Positionsdaten mit den in einer Nachschlagetabelle gespeicherten Positionsdaten, zum Auslesen von Kalibrierungsdaten, die unter zumindest im wesentlichen mit den ermittelten Positionsdaten übereinstimmenden Positionsdaten in der Nachschlagetabelle gespeichert sind, sowie zum Entzerren des aufgenommenen Bildes des Untersuchungsobjektes oder zum Verzerren des Bildes eines in das Untersuchungsobjekt eingeführten Instrumentes mit den ausgelesenen und erforderlichenfalls interpolierten Kalibrierungsdaten; und einer Anzeigeeinheit zur Darstellung des entzerrten Bildes des Untersuchungsobjektes oder zur Darstellung des verzerrten Bildes des Untersuchungsobjektes, in das das verzerrte Bild des Instrumentes eingeblendet ist.

Vorteile dieser Lösungen bestehen in erster Linie darin, dass damit wesentlich bessere Bildqualitäten erzielt werden können, dass die Handhabung einfach ist und dass sie auch zur Anwendung mit mobilen Röntgensystemen geeignet sind, ohne dass die üblichen Positionsmesssysteme erforderlich sind.

Schließlich können damit auch Verzerrungen korrigiert werden, die durch eine gekrümmte Oberfläche des Eintrittsfensters des Bildverstärkers entstehen.

Die Unteransprüche haben vorteilhafte Weiterbildungen der Erfindung zum Inhalt.

Die Kalibrierungsdaten können im einfachsten Fall gemäß Anspruch 2 durch Aufnehmen eines Phantomobjektes oder gemäß Anspruch 3 mit Hilfe eines physikalischen Modells berechnet werden.

Die Ausführung gemäß Anspruch 4 bietet sich insbesondere bei schwenkbaren Tragekonstruktionen an.

Die Ausführung gemäß Anspruch 7 ist schließlich insbesondere für die chirurgische Navigation geeignet.

Weitere Einzelheiten, Merkmale und Vorteile der Erfindung ergeben sich aus der folgenden Beschreibung von bevorzugten Ausführungsformen anhand der Zeichnung Es zeigt:
Fig 1 eine schematische Gesamtansicht eines Röntgensystems mit einer erfindungsgemäßen Vorrichtung; und
Fig 2 eine schematische Darstellung einer Anordnung zur Kalibrierung des Röntgensystems.

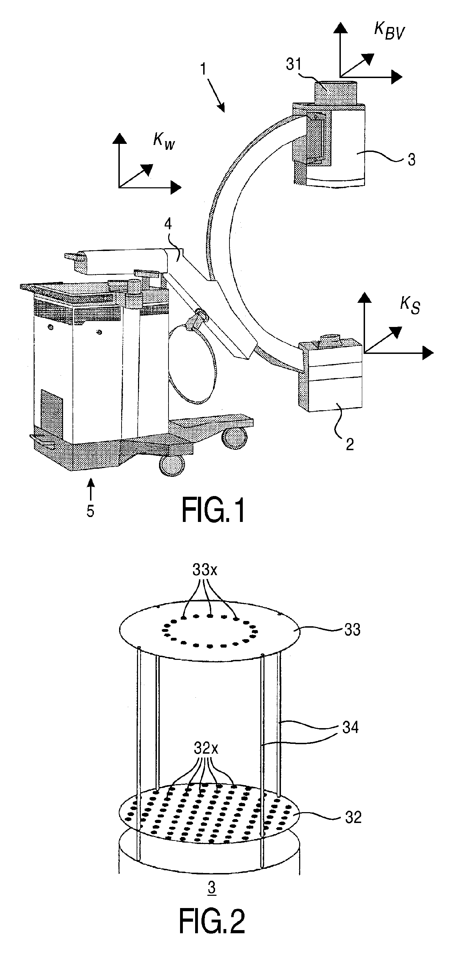

Figur 1 zeigt ein mobiles Röntgensystem mit einem C-Arm 1, an dessen einem Ende eine Röntgenstrahlenquelle 2 und an dessen gegenüberliegendem Ende ein Röntgenstrahlendetektor 3 mit Bildverstärker angeordnet ist. An dem C-Arm 1 ist außerdem ein Drei-Achsen-Beschleunigungsmesser 31 befestigt, mit dem die Richtung des Schwerkraftfeldes relativ zu dem C-Arm 1 gemessen wird. Der C-Arm 1 ist schwenkbar an einer Halterung 4 gelagert, die wiederum an einem Tisch 5 befestigt ist. Der Tisch 5 ist fahrbar und mit Bedienungselementen sowie Versorgungs- und Betriebseinrichtungen für das Röntgensystem versehen.

Zwischen die Quelle 2 und den Detektor 3 wird ein Untersuchungsobjekt (Patient) eingebracht, wobei der C-Arm 1 an der Halterung 4 im allgemeinen um mindestens 180 Grad geschwenkt werden kann, um eine optimale Durchleuchtung des zu untersuchenden Bereiches erzielen zu können.

Wie eingangs bereits erwähnt wurde, kann ein solcher C-Arm durch das Gewicht der Röntgenstrahlenquelle 2 und des Röntgenstrahlendetektors 3 sowie des Bildverstärkers deformiert werden, so dass aufgenommene Bilder verzerrt sind. Aus diesem Grund ist eine Kalibrierung durchzuführen, mit der die von der Schwenkstellung des C-Arms abhängigen Deformationen kompensiert und somit die dadurch verursachten unterschiedlichen Verzerrungen korrigiert werden.

Die Kalibrierung erfolgt nach der Herstellung des Röntgensystems sowie gegebenenfalls in regelmäßigen Zeitabständen (Serviceintervalle). Die wesentlichen Schritte eines solchen Verfahrens sollen im folgenden erläutert werden. Alternativ dazu sind jedoch auch Abwandlungen dieses Verfahrens oder andere Verfahren anwendbar.

Zunächst wird gemäß der Darstellung in Figur 2 ein Phantomobjekt in Form von zwei parallelen Platten 32, 33 an dem Röntgenstrahlendetektor 3 befestigt. Zu diesem Zweck dienen Stangen 34, mit denen die Platten auch parallel gehalten werden. Die erste Platte 32 liegt direkt vor dem Eintrittsfenster des Detektors 3 und ist mit einer Mehrzahl von Kreisflächen oder Kugeln 32x versehen, die auf den Gitterpunkten eines gedachten quadratischen Gitters angsordnet und für Röntgenstrahlen undurchlässig sind. Zwischen der ersten Platte 32 und der Röntgenstrahlenquelle 2 befindet sich die zweite Platte 33 mit einem Abstand von zum Beispiel etwa 37 Zentimeter von der ersten Platte. Die zweite Platte ist mit einer Mehrzahl von genau so großen und für Röntgenstrahlen ebenfalls undurchlässigen Kreisflächen oder Kugeln 33x versehen, die jedoch auf dem Umfang eines zentrierten Kreises angeordnet sind

Die erste Platte 32 dient zur Ermittlung der Verzerrungsparameter eines Projektionsbildes, während die zweite Platte 33 zur Ermittlung der tatsächlichen Brennpunktsposition dient, und zwar jeweils für eine Vielzahl von gewählten Schwenkstellungen des C-Arms 1. Die ermittelten Verzerrungsparameter und Brennpunktspositionen werden als Verzerrungs-Datensätze für jede der Schwenkstellungen des C-Arms 1 abgespeichert.

Im einzelnen werden die Kreisflächen oder Kugeln 32x der ersten Platte 32 auf den Detektor 3 projiziert, mit einem Segmentierungsalgorithmus erfasst und den einzelnen Kreisflächen oder Kugeln 32x auf der ersten Platte, deren Positionen bekannt sind, zugeordnet. Dadurch sowie durch entsprechende Interpolation zwischen den Flächen können dann die Verzerrungsparameter für "jeden" Röntgenstrahl und somit für jeden Bildpunkt in an sich bekannter Weise berechnet werden.

Weiterhin wird mit Hilfe des durch die zweite Platte 33 auf den Detektor projizierten Bildes des Kreises von Kreisflächen 33x und des Verhältnisses zwischen den Durchmessern dieses Kreises sowie des projizierten Kreises die Brennpunktsposition in an sich bekannter Weise berechnet.

Aus den für jede der Schwenkstellungen des C-Arms 1 aufgenommenen Verzerrungs-Datensätzen werden dann Kalibrierungsdaten (Kalibrierungs-Basispunkte) berechnet, die abgespeichert werden und mit denen die durch die Verzerrungen und Brennpunktsverschiebungen verursachten Fehler eines in der betreffenden Schwenkstellung aufgenommenen Bildes korrigiert (entzerrt) werden können.

Weiterhin wird mit dem Drei-Achsen-Beschleunigungsmesser 31 in jeder dieser Schwenkstellungen des C-Arms 1 das Schwerkraftfeld im Hinblick auf seine Richtung relativ zu dem C-Arm gemessen. Aus dieser Richtung werden dann darauf bezogene Positionsdaten des C-Arms, inbesondere dessen tatsächlicher Schwenkwinkel bzw. dessen Drehstellung berechnet.

Diese Positionsdaten werden schließlich zusammen mit den für diese Stellung ermittelten Kalibrierungsdaten in einander zugeordneter Weise in einer Nachschlagetabelle gespeichert.

Wenn für eine ausreichende Anzahl von Schwenkstellungen des C-Arms 1 Kalibrierungsdaten sowie die Positionsdaten dazu ermittelt und in der Nachschlagetabelle abgespeichert wurden, ist die Kalibrierung des Röntgensystems abgeschlossen.

Alternativ dazu könnte die Nachschlagetabelle auch auf der Grundlage eines physikalischen Modells der mechanischen Deformation des C-Arms in verschiedenen Stellungen sowie der jeweils zur Korrektur erforderlichen Kalibrierungsdaten berechnet werden.

Zur Kalibrierung, d. h zur Erstellung und Abspeicherung der Nachschlagstabelle, ist entweder eine gesonderte Rechen- und Speichereinheit vorgssehen, oder die Kalibrierung wird durch ein entsprechendes Datenverarbeitungsprogramm mit einer in dem betreffenden Röntgensystem vorhandenen Rechnereinheit vorgenommen.

Zur Bilderzeugung bei der Untersuchung eines Patienten oder eines anderen Objektes wird der C-Arm 1 zunächst wie üblich in eine Position geschwenkt, in der der interessierende Bereich durchleuchtet und ein entsprechendes Abbild auf den Bildverstärker projiziert werden kann. Wenn diese Position erreicht ist, wird das Bild in bekannter Weise aufgenommen. Weiterhin wird mit dem Drei-Achsen-Beschleunigungsmesser 31 das Schwerkraftfeld im Hinblick auf seine Richtung relativ zu dem C-Arm erfasst. Aus dieser relativen Richtung des Schwerkraftfeldes werden dann wiederum die Positionsdaten des C-Arms 1 berechnet und mit den in der Nachschlagetabelle gespeicherten Positionsdaten verglichen. Wenn ein übereinstimmender oder im wesentlichen übereinstimmender Eintrag gefunden werden, werden die diesem Eintrag zugeordneten Kalibrierungsdaten ausgelesen und zur Korrektur des aufgenommenen Bildes in an sich bekannter Weise verwendet.

Alternativ dazu können die Positionsdaten des C-Arms bei der Kalibrierung und der Bilderzeugung zum Beispiel auch mit einem optischen Positionssmesssystem (OPMS) berechnet werden können, so dass der Beschleunigungsmesser nicht erforderlich ist. Weiterhin könnte der Beschleunigungsmesser auch an einem die Schwenkstellung des C-Arms verfolgenden System befestigt sein, um aus dessen Stellung die Positionsdaten des C-Arms zu berechnen.

Wenn keine ausreichende Übereinstimmung zwischen den ermittelten und den in der Tabelle gespeicherten Positionsdaten gefunden wird, müssen die betreffenden Kalibrierungsdaten interpoliert werden. Zu diesem Zweck seinen die Kalibrierungsdaten als Kalibrierungs-Basispunkte betrachtet. Es sind verschiedene Verfahren anwendbar. Beispielhaft sei hier die Anwendung einer genäherten Delauney Triangulation erläutert.

Es handelt sich dabei deshalb um eine Näherung der Triangulation, weil die Kalibrierungs-Basispunkte auf einer kugelförmigen Fläche liegen, aus Gründen der Einfachheit die durch die Basispunkte gebildeten Delauney-Dreiecke jedoch als planare Dreiecke behandelt werden sollen. Vor der Interpolation muss deshalb jeder Basispunkt auf solche planaren Dreiecke projiziert werden.

Das ursprüngliche (planare) Verfahren arbeitet wie folgt. Es sei von einem Satz von zu interpolierenden Kalibrierungs-Baaspunkten auf einer Kugelfläche ausgegangen. Der Triangulations-Algorithmus führt zu einem Satz von sich nicht schneidenden ebenen Dreiecken, deren Ecken jeweils durch Kalibrierungs-Basispunkte gebildet sind, so dass die gesamte Fläche mit Dreiecken bedeckt ist.

Jeder beliebige Zwischenpunkt P (d. h ein zu interpolierender Kalibrierungs-Basispunkt) kann nun eindeutig einem der Dreiecke zugeordnet werden. Die Eckpunkte dieses Dreiecks stellen die drei Kalibrierungs-Basispunkte dar, die dem Punkt P am nächsten liegen. Somit sind die Basispunkte bestimmt, die für die Interpolation zu wählen sind.

Die Delauney Triangulation ist eindeutig Für eine zweidimensionale Ebene arbeitet der Algorithmus wie folgt: zunächst werden aus dem Satz von Kalibrierungs-Basispunkten alle möglichen Dreiecke gebildet. Diejenigen Dreiecke, deren Eckpunkte kollinear sind, bleiben unberücksichtigt. Wenn der ein Dreieck umschreibende Kreis andere Basispunkte enthält, bleiben das Dreieck ebenfalls unberücksichtigt. Nur wenn diese beiden Fälle nicht gegeben sind, wird das Dreieck verwendet.

Da die Kalibrierungs-Basispunkte jedoch tatsächlich auf einer Kugelfläche liegen, wird zur Vermeidung von geometrischen Verzerrungen und anderen Problemen die Delauney Triangulation an eine sphärische Interpolation angepasst und in der Weise geändert (genähert), dass die Kalibrierungs-Basispunkte in ein dreidimensionales kartesisches Koordinatensystem transformiert werden. Dabei bleibt für jedes Tripel von koplanaren Basispunkten das entsprechende Dreieck unberücksichtigt.

Anstelle des oben genannten umschreibenden Kreises wird eine umschließende Kugel gebildet, und deren Radius wird mit dem dreidimensionalen euklidischen Abstand von jedem anderen Kalibrierungs-Basispunkt verglichen. Man kann nachweisen, dass dieses Kriterium äquivalent zu dem der normalen zweidimensionalen Delauney Triangulation ist, wenn die Basispunkte auf einer idealen Kugelfläche liegen. Wenn die umschließende Kugel anderer Basispunkte enthält, bleibt das Dreieck unberücksichtigt. Wenn dies nicht der Fall ist, ist das Dreieck anwendbar.

Zur Vereinfachung sowie zur Beschleunigung der Interpolationsberechnungen wird anstelle des auf der Kugelfläche liegenden Punktes P die Projektion P' des Punktes P auf die ebene Dreiecksfläche betrachtet. Dies führt nur bei großen Dreiecken zu geringfügigen Verzerrungseffekten bei den Interpolations-Beiträgen.

Wenn die Triangulation abgeschlossen ist, kann die Interpolation durch die planaren Dreiecke in einfacher Weise unter Anwendung von Schwerpunkts-Koordinaten (baryzentrische Koordinaten) berechnet werden. Ein in der Ebene (C1, C2, C3) liegender Punkt P' kann durch seine baryzentrischen Koordinaten (B1, B2, B3) beschrieben werden. Im folgenden sei angenommen, dass ein Punkte P auf der Kugelfläche, für den die Interpolations-Koeffizienten zu berechnen sind, auf jede zu berücksichtigende planare Dreieckfläche projiziert wird (Punkt P').

Die baryzentrischen Koordinaten beinhalten jeweils eine Information über die relative Position des Punktes P' in Bezug auf eine der Seiten des Dreiecks. Für einen Eckpunkt C1 des Dreiecks ist B1 negativ, wenn der Punkt P' jenseits der durch die Eckpunkte C2 und C3 verlaufenden Linie liegt, 0 wenn er auf dieser Linie liegt und positiv, wenn er sich auf der gleichen Seite der Linie befindet, wie der Eckpunkt C1.

Auf diese Weise bieten die baryzentrischen Koordinaten ein einfaches Kriterium für das Auffinden des geeigneten Interpolations-Dreiecks. Nur wenn alle Werte Bi größer als 0 sind, liegt der Punkt P' innerhalb des Dreiecks.

Nachdem nun die baryzentrischen Koordinaten ermittelt wurden, können die für den Punkt P' zu interpolierenden Werte durch eine einfache lineare Kombination ermittelt und somit der interpolierte Kalibrierungs-Basispunkt berechnet werden, mit dem dann das aufgenommene Bild korrigiert bzw. entzerrt wird.

Zur Bilderzeugung und eine gegebenenfalls erforderliche Interpolation ist wiederum entweder eine gesonderte Rechen- und Speichereinheit oder ein entsprechendes Datenverarbeitungsprogramm vorgesehen, das mit einer in dem betreffenden Rontgensystem vorhandenen Rechnereinheit ausgeführt wird

Ergänzend sei darauf hingewiesen, dass neben der Bildentzerrung eines aufgenommenen Bildes das erfindungsgemäße Prinzip zum Beispiel auch zur chirurgischen Navigation anwendbar ist. Dabei kommt es nicht in erster Linie darauf an, ein aufgenommenes Bild zu entzerren, sondern möglichst genau die Position eines in den Patienten eingeführten Instrumentes (zum Beispiel eines Katheters) zu ermitteln und mit einem entsprechenden Bildverarbeitungssystem in das aufgenommene Bild einzublenden.

Dabei wird einerseits in üblicher Weise ein Röntgenbild des zu untersuchenden Bereiches eines Patienten aufgenommen, ohne dieses zu entzerren. Andererseits wird die aktuelle Position des eingeführten Instruments mit Hilfe eines bekannten Verfahrens oder eines Positionsmessgerätes (zum Beispiel mittels eines kleinen Senders oder einer Induktivität an der Spitze des Instruments) laufend ermittelt. Diese Position wird dann unter (umgekehrter) Anwendung der Nachschlagetabelle, die die Verzerrungseingenschaften des Röntgengerätes beschreibt, verzerrt. Anders ausgedrückt bedeutet dies, dass das (virtuelle) Bild des eingeführten Instruments entsprechend den in Form der Kalibrierungsdaten gespeicherten Abbildungseigenschaften des Röntgengerätes verzerrt wird. Dieses verzerrte Bild wird dann unter Anwendung einer entsprechenden Anzeigeeinheit in das aufgenommene (verzerrte) Röntgenbild eingeblendet, so dass das Instrument nun an der richtigen Stelle in dem Röntgenbild erscheint.

Dies hat den Vorteil, dass auch bei einer laufenden Verfolgung bzw. laufend zu aktualisierenden Darstellung des (im allgemeinen geführten) Instrumentes nur eine Röntgenaufnahme gemacht werden muss. Außerdem braucht dabei das Instrument noch nicht eingeführt zu sein, so dass die Röntgenaufnahme dadurch auch nicht beeinträchtigt werden kann.

Eine Entzerrung des Bildes ist dabei nicht notwendig, da nur die tatsächliche Position des Instrumentes relativ zu dem zu untersuchenden Objekt von Bedeutung ist.

## Patentansprüche

1. Verfahren zur Kalibrierung einer mit einer schwerkraftempfindlichen Tragekonstruktion gehaltenen Bildaufnahmeeinrichtung mit folgenden Schritten:
- Ermitteln von Kalibrierungsdaten in einer Mehrzahl von gewählten Stellungen der Tragekonstruktion, mit denen ein in der jeweiligen Stellung mit der Bildaufnahmeeinrichtung aufgenommenes Bild entzerrt werden kann;
- Ermitteln von jeweils auf die Richtung der Schwerkraft bezogenen Positionsdaten der Tragekonstruktion in den gewählten Stellungen; und
- Zuordnen der Positionsdaten zu den Kalibrierungsdaten in einer Nachschlagetabelle.

2. Verfahren nach Anspruch 1,
**dadurch gekennzeichnet,**
**dass** die Kalibrierungsdaten mit Hilfe eines aufgenommenen Phantomobjektes ermittelt werden.

3. Verfahren nach Anspruch 1,
**dadurch gekennzeichnet,**
**dass** die Kalibrierungsdaten mit Hilfe eines physikalischen Modells der mechanischen Deformation der Tragekonstruktion berechnet werden.

4. Verfahren nach Anspruch 1,
**dadurch gekennzeichnet,**
**dass** die Positionsdaten die Schwenkwinkel der Tragekonstruktion sind.

5. Vorrichtung zur Durchführung des Verfahrens nach einem der Ansprüche 1 bis 4, mit
- einer Einrichtung (31) zum Erfassen der Richtung der Schwerkraft relativ zu einer gewählten Stellung der Tragekonstruktion; und
- einer Rechen- und Speichereinheit zum Ermitteln von auf die Richtung der Schwerkraft bezogenen Positionsdaten für eine Mehrzahl von gewählten Stellungen der Tragekonstruktion und zum Erstellen und Abspeichern einer Nachschlagstabelle, mit der den Positionsdaten jeweils die zur Entzerrung von in diesen Stellungen durch mechanische Deformationen verursachten Bildverzerrungen geeigneten Kalibrierungsdaten zugeordnet sind.

6. Verfahren zur Bilderzeugung mit einer mit einer schwerkraftempfindlichen Tragekonstruktion gehaltenen Bildaufnahmeeinrichtung, die insbesondere nach einem der Ansprüche 1 bis 4 kalibriert ist, mit folgenden Schritten:
- Aufnehmen eines Bildes eines Untersuchungsobjektes in einer gewählten Stellung der Tragekonstruktion;
- Ermitteln von auf die Richtung der Schwerkraft bezogenen Positionsdaten der Tragekonstruktion in der gewählten Stellung;
- Vergleichen der ermittelten Positionsdaten mit den in einer Nachschlagetabelle gespeicherten Positionsdaten;
- Auslesen von Kalibrierungsdaten, die unter zumindest im wesentlichen mit den ermittelten Positionsdaten übereinstimmenden Positionsdaten in der Nachschlagetabelle gespeichert sind; und
- Entzerren des aufgenommenen Bildes mit den ausgelesenen Kalibrierungsdaten.

7. Verfahren zur Bilderzeugung insbesondere bei der chirurgischen Navigation mit einer mit einer schwerkraftempfindlichen Tragekonstruktion gehaltenen Bildaufnahmeeinrichtung, die insbesondere nach einem der Ansprüche 1 bis 4 kalibriert ist, mit folgenden Schritten: Aufnehmen eines Bildes eines Untersuchungsobjektes in einer gewählten Stellung der Tragekonstruktion;
- Ermitteln von auf die Richtung der Schwerkraft bezogenen Positionsdaten der Tragekonstruktion in der gewählten Stellung;
- Vergleichen der ermittelten Positionsdaten mit den in einer Nachschlagetabelle gespeicherten Positionsdaten;
- Auslesen von Kalibrierungsdaten, die unter zumindest im wesentlichen mit den ermittelten Positionsdaten übereinstimmenden Positionsdaten in der Nachschlagetabelle gespeichert sind;
- Ermitteln der Position und Aufnehmen eines (virtuellen) Bildes eines in das Untersuchungsobjekt eingeführten Instrumentes;
- Berechnen eines virtuellen, verzerrten Bildes des in das Untersuchungsobjekt eingeführten Instrumentes mit Hilfe der ausgelesenen Kalibrierungsdaten; und
- Einblenden des verzerrten Bildes des eingeführten Instrumentes in das aufgenommene Bild des Untersuchungsobjektes.

8. Datenverarbeitungsprogramm mit Programmcode-Mitteln zur Durchführung der Schritte des Verfahrens nach einem der Ansprüche 1 bis 4 oder 6 oder 7, wenn das Programm in einem Computer ausgeführt wird.

9. Vorrichtung zur Durchführung des Verfahrens nach Anspruch 6 oder 7, mit
- einer Einrichtung (31) zur Erfassung der Richtung der Schwerkraft relativ zu der Tragekonstruktion;
- einer Rechen- und Speichereinheit zum Ermitteln von auf die Richtung der Schwerkraft bezogenen Positionsdaten der Tragekonstruktion, zum Vergleichen dieser Positionsdaten mit den in einer Nachschlagetabelle gespeicherten Positionsdaten, zum Auslesen von Kalibrierungsdaten, die unter zumindest im wesentlichen mit den ermittelten Positionsdaten übereinstimmenden Positionsdaten in der Nachschlagetabelle gespeichert sind, sowie zum Entzerren des aufgenommenen Bildes des Untersuchungsobjektes oder zum Verzerren des Bildes eines in das Untersuchungsobjekt eingeführten Instrumentes mit den ausgelesenen und erforderlichenfalls interpolierten Kalibrierungsdaten, sowie
- einer Anzeigeeinheit zur Darstellung des entzerrten Bildes des Untersuchungsobjektes oder zur Darstellung des verzerrten Bildes des Untersuchungsobjektes, in das das verzerrte Bild des Instrumentes eingeblendet ist.

10. Röntgensystem mit einem Bildverstärker und einer Vorrichtung nach Anspruch 5 oder 9.
